# EUROPÄISCHE PATENTANMELDUNG

(11) **EP 3 809 260 A1**
(43) Veröffentlichungstag der Anmeldung: **21.04.2021**
(21) Anmeldenummer: 19203988.1
(22) Anmeldetag: 18.10.2019
(51) Int. Cl.: G06F 9/445, A61B 5/00, A61B 6/00, A61B 8/00

(54) **SYSTEM UND VERFAHREN ZUR ADMINISTRATION VON BILDGEBENDEN GERÄTEN**

(71) Anmelder: Siemens Healthcare GmbH, 91052 Erlangen (DE)
(72) Erfinder: Berreth, Marc, 91058 Erlangen (DE); Christiani, Peter, 97447 Gerolzhofen (DE); Hoecker, Ludwig, 90542 Eckental (DE); Ortmüller, Jonas, 91058 Erlangen (DE); Sawinsky, Kristina, 96155 Buttenheim (DE); Schor, Stefan, 91056 Erlangen (DE)

(57) **Zusammenfassung**

Die Erfindung betrifft ein System zur Administration einer Gruppe von bildgebenden Geräten, die jeweils mit einem Geräte-Host ausgestattet sind. Auf dem Geräte-Host sind eine Geräte-Software und ein Konfigurationsprotokoll ausführbar gespeichert. Weiterhin umfasst das System ein verteiltes Datenbanksystem, welches zum Empfangen von Konfigurationsprotokollen von dem Geräte-Host und zum Speichern von geänderten Konfigurationsprotokollen für den jeweiligen Geräte-Host konfiguriert ist, sowie geänderte Konfigurationsprotokollen zu verteilen. Über einen zentralen Schreibzugriff werden die empfangenen und/oder gespeicherten Konfigurationsprotokolle bereitgestellt. Weiterhin umfasst das System eine Administratoreinheit mit einem Speicher, einer Benutzerschnittstelle und einem Prozessor zur Kommunikation mit dem verteilten Datenbanksystem. Über die Benutzerschnittstelle können Konfigurationsprotokolle geändert werden, welche an das verteilte Datenbanksystem zur Speicherung und/oder Verteilung an wenigstens ein ausgewähltes bildgebendes Gerät gesendet werden. Die Erfindung betrifft ferner ein Verfahren zur Administration von bildgebenden Geräten.

## Beschreibung

Die Erfindung betrifft ein System zur Administration von bildgebenden Geräten, wie z.B. von einer Flotte von MR-Scannern. Ferner betrifft die Erfindung ein Verfahren zur Administration der bildgebenden Geräten, sowie ein Computerprogramm.

Bildgebende Geräte werden über eine Geräte-Software, die auf einem Geräte-Host betrieben wird, angesteuert. Die Geräte-Hosts stellen isolierte Rechner dar, die nur für einen Stand-Alone-Betrieb vorgesehen sind. Daher besteht keine Möglichkeit, vorgenommene Änderungen an Konfigurationsprotokollen eines Geräte-Hosts an einem (anderen) bildgebenden Gerät zu erkennen und/oder in die Gruppe zu verteilen.

Lösungen zur Pflege, Aktualisierung und/oder zum Verteilen von geänderten Konfigurationsprotokollen sehen einen manuellen und lokalen Zugriff auf den Geräte-Host des entsprechenden bildgebenden Gerätes vor. Um diesbezüglich ein geändertes Konfigurationsprotokoll eines Geräte-Hosts auf einen anderen Geräte-Host zu übertragen, werden diese mittels eines externen Datenträgers, beispielsweise einer portablen Festplatte, CD, DVD oder eines USB-Sticks manuell durch einen Anwender von einem Geräte-Host exportiert und anschließend manuell auf einen anderen Geräte-Host importiert. Da jedes bildgebende Gerät spezifische Eigenschaften, beispielsweise die Hardware-Konfiguration und/oder Lizenzeigenschaften aufweist, ist nach dem Importieren des geänderten Konfigurationsprotokolls eine Konvertierung der Konfigurationsprotokolle notwendig, um eine entsprechende Kompatibilität des geänderten Konfigurationsprotokoll zu dem neuen bildgebenden Gerät zu erreichen.

Die bekannten Lösungen stellen eine ineffiziente Möglichkeit zur Änderung von Konfigurationsprotokollen, insbesondere zur Verteilung von geänderten Konfigurationsprotokollen dar, da ein manueller und vor Ort durchgeführter und somit zeitaufwendiger Export und Import der Konfigurationsprotokolle erfolgen muss. Zudem ist eine Konvertierung der Konfigurationsprotokolle für das aufnehmende Host-Gerät entsprechend der Eigenschaften des bildgebenden Gerätes notwendig. Die genannten Schritte beinhalten ein enormes Fehlerpotential, welches die Zuverlässigkeit der bildgebenden Geräte und somit deren Einsatzzeiten minimieren kann. Zudem ist während des Imports der Konfigurationsprotokolle und des Vorganges zum Konvertieren der Konfigurationsprotokolle eine Nutzung des bildgebenden Gerätes nicht möglich, so dass dieses außer Betrieb gehen muss.

Es besteht daher ein Bedarf an einem verbesserten Mechanismus zur Administration von bildgebenden Geräten. Ausgehend vom aufgezeigten Stand der Technik und dem sich daraus ergebenden Bedarf, hat sich die vorliegende Erfindung zur Aufgabe gestellt, eine Lösung zu schaffen, die die im Stand der Technik bekannten Nachteile zu mindestens teilweise überwindet.

Diese Aufgabe wird durch die Merkmale des Anspruchs 1, insbesondere durch ein System zur Administration von bildgebenden Geräten gelöst, sowie durch die nebengeordneten beiliegenden Ansprüche, insbesondere durch ein Verfahren und ein Computerprogramm.

Gemäß einem ersten Aspekt betrifft die vorliegende Erfindung ein System zur Administration von bildgebenden Geräten. Das System umfasst mindestens ein bildgebendes Gerät. Vorzugsweise umfasst das System eine Vielzahl an bildgebenden Geräten. Die Vielzahl der bildgebenden Geräte können einer Flotte oder Gruppe von bildgebenden Geräten zugeordnet sein. Einer Gruppe können bildgebende Geräte mit unterschiedlichen und/oder gleichen spezifischen Eigenschaften zugeordnet werden. Spezifische Eigenschaften sind technische Geräteeigenschaften des MR-Scanners oder des CT-Gerätes oder eines anderen bildgebenden Gerätes, wie z.B. Anzahl der Spulen, aufgespielte Software-Version zum Betrieb des Gerätes, eingerichtete Bauteile und Komponenten sowie Lizenzen.

Im Sinne der vorliegenden Erfindung umfasst eine Administration einen technischen Betrieb der bildgebenden Geräte und/oder eine Ausführung von Maßnahmen zum Betreiben der bildgebenden Geräte.

Das bildgebende Gerät umfasst einen Geräte-Host. Der Geräte-Host ist als eine Recheneinheit mit einem flüchtigen und/oder nichtflüchtigen Speicher, wenigstens einem Prozessor und Kommunikationsschnittstellen zur Kommunikation mit dem bildgebenden Gerät und zur Kommunikation mit einem Anwender des bildgebenden Gerätes ausgebildet. Ferner kann der Geräte-Host Kommunikationsschnittstellen zur Kommunikation mit weiteren Geräten aufweisen. Die Kommunikationsschnittstellen können für eine kabelgebundene und/oder kabellose Kommunikation ausgebildet sein. Insbesondere kann der Geräte-Host als ein Computer, ein Laptop, eine Workstation etc. ausgebildet sein. Der Geräte-Host ist konfiguriert, eine Geräte-Software, ein sogenanntes Binary, zum Bedienen und Ansteuern des bildgebenden Gerätes zu speichern und auszuführen.

Die Geräte-Software weist eine spezifische Software-Version auf. Eine Software-Version stellt hierbei ein definiertes Entwicklungsstadium der Software des bildgebenden Gerätes mit allen dazugehörigen Komponenten dar. Verschiedene Versionen stellen die Veränderung und Weiterentwicklung einer Software oder eines Teils über eine bestimmte Zeit dar, welche eine gemeinsame historische Basis aufweisen. Die historische Basis umfasst die Major-Version. So können Systeme zur Versionierung genutzt werden, um neuere Ausgaben einer Software von einer älteren zu unterscheiden.

Ferner ist zumindest ein Konfigurationsprotokoll in dem Speicher des Geräte-Hosts ausführbar gespeichert. Ein Konfigurationsprotokoll unterscheidet sich hinsichtlich der verfügbaren Hardware des bildgebenden Gerätes, auf dem es zum Einsatz kommt. Jedes bildgebende Gerät kann entsprechend des Einsatzspektrums in Funktion und Anzahl unterschiedliche Geräte-Komponenten aufweisen, welche unterschiedlich angesprochen und/oder konfiguriert werden können/müssen. Diese Geräte-Komponenten bzw. die Parameter der Geräte-Komponenten werden über die Konfigurationsprotokolle eingestellt und konfiguriert. Das Einstellen und Konfigurieren können beispielsweise ein Auswählen der Leistung, Ströme, Auflösung etc. umfassen. Weiterhin kann sich das Konfigurationsprotokoll bezüglich verfügbarer Lizenzen zum Betrieb des bildgebenden Gerätes und/oder von Funktionalitäten des bildgebenden Gerätes unterscheiden. Bildgebende Geräte bei unterschiedlichen Anwendern können in ihrer Hardware und/oder ihrem Softwareumfang gleich ausgestaltet sein, allerdings wird der Funktionsumfang der Hardware und/oder Software über ein Lizenzmodell bestimmt. So kann nur der Funktionsumfang, für den entsprechenden Lizenzen erworben wurden, auf dem Gerät verwendet werden. Diesbezüglich wird in dem entsprechenden Konfigurationsprotokoll nur der Funktionsumfang berücksichtigt, für welchen Lizenzen zur Verfügung stehen.

Ferner weist das System ein verteiltes Datenbanksystem auf. Das verteilte Datenbanksystem kann eine Datenbank und/oder eine Vielzahl an Datenbanken als ein zentrales Speichermedium umfassen. Die Datenbank kann Konfigurationsprotokolle effizient, widerspruchsfrei und dauerhaft speichern und benötigte Konfigurationsprotokolle bedarfsgerecht bereitstellen. Die Datenbank kann beispielsweise als eine Oracle Datenbank oder MYSQL-Datenbank ausgeführt sein. Weitere Ausgestaltungen, die die Anforderungen an das verteilte Datenbanksystem der vorliegenden Erfindung erfüllen, können ebenfalls eingesetzt werden. Das Datenbanksystem kann auf einem lokalen Server oder einem Serververbund oder gehostet in einer Cloud bereitgestellt werden. Das verteilte Datenbanksystem weist Kommunikationsschnittstellen für einen Fernzugriff von dem Geräte-Host des bildgebenden Gerätes auf das verteilte Datenbanksystem auf. Das verteilte Datenbanksystem ist konfiguriert, Konfigurationsprotokolle von dem Geräte-Host zu empfangen und für den jeweiligen Geräte-Host zu speichern und geänderte Konfigurationsprotokolle zu verteilen und einen zentralen Schreibzugriff auf die empfangenen und/oder gespeicherte Konfigurationsprotokolle bereitzustellen. Über den zentralen Schreibzugriff kann eine Administratoreinheit auf das verteilte Datenbanksystem zugreifen.

Das verteilte Datenbanksystem bildet eine Systemstruktur ab, die anfragenden Programmen und/oder Geräten über eine Schnittstelle einen Zugriff ermöglicht. Das verteilte Datenbanksystem ermöglicht anfragenden Programmen und/oder Geräten den Zugriff auf unterschiedliche, verteilte Datenbanken, die nach außen hin als eine einheitliche Datenbank erscheinen können, aber z.B. intern für die jeweiligen Hosts zugeordnet und strukturiert sind.

Ferner weist das System eine Administratoreinheit auf. Die Administratoreinheit kann als ein Computer oder Handheld, beispielsweise ein Laptop, Tablet, Smartphone usw. ausgebildet sein. Die Administratoreinheit umfasst einen flüchtigen und/oder nichtflüchten Speicher, beispielsweise RAM, ROM, Flash, Festplattenspeicher zum lokalen Speichern einer Firmware, eines Betriebssystems, einer Anwendungssoftware und/oder von Anwendungsdaten, beispielsweise Konfigurationsprotokollen.

Weiterhin umfasst die Administratoreinheit eine Benutzerschnittstelle, beispielsweise ein Display, auf der eine Interaktion zwischen einem Anwender und der Administratoreinheit erfolgen kann. Eine Interaktion kann beispielsweise ein Anzeigen von Konfigurationsprotokollen, beispielsweise zur Analyse und/oder eine Eingabe von Änderungen an Konfigurationsprotokollen umfassen. Die Interaktion wird über eine auf der Administratoreinheit installierte Applikation bzw. Anwendungssoftware gesteuert.

Ferner weist die Administratoreinheit Kommunikationsschnittstellen zur operativen Kommunikation eines Prozessors mit dem verteilten Datenbanksystem auf. Die Kommunikationsschnittstellen können als kabelgebundene Kommunikationsschnittstellen, beispielsweise Ethernet oder als kabellose Kommunikationsschnittstellen, beispielsweise WLAN ausgebildet sein. In dem Speicher der Administratoreinheit kann eine Anwendungssoftware zum Verwalten und/oder Bearbeiten von lokal gespeicherten Konfigurationsprotokollen gespeichert sein, die durch einen Prozessor der Administratoreinheit ausgeführt wird. In einer Ausführungsform kann die Administratoreinheit eine Vielzahl an Prozessoren gegebenenfalls mit einer Vielzahl an Prozessorkernen aufweisen. In einer alternativen Ausführungsform kann die Administratoreinheit eine Anwendungssoftware zum Zugriff auf webbasierte Inhalte gespeichert bereitstellen, mittels der webbasiert Konfigurationsprotokolle verwaltet und/oder bearbeitet werden können.

Die Administratoreinheit ist dazu konfiguriert, von dem verteilten Datenbanksystem zumindest ein gespeichertes Konfigurationsprotokoll für einen Gerät-Host eines bildgebenden Gerätes zu empfangen und dieses gegebenenfalls und insbesondere bei Änderungsintention lokal in dem Speicher der Administratoreinheit zu speichern. Die Administratoreinheit ist ausgebildet, insbesondere in Antwort auf einen Administrationsbefehl für jedes bildgebende Gerät der Flotte/Gruppe ein auf dem verteilten Datenbanksystem gespeichertes Kommunikationsprotokoll zu empfangen und dieses lokal in dem Speicher der Administratoreinheit zu speichern.

Über die Benutzerschnittstelle der Administratoreinheit können die gespeicherten Konfigurationsprotokolle zentral geändert werden. Weiterhin kann über die Benutzerstelle ein geändertes Konfigurationsprotokoll erzeugt werden. Die Benutzerschnittstelle kann als eine Anwendungssoftware ausgebildet sein, die auf einem Display der Administratoreinheit ausgegeben wird. Ein Konfigurationsprotokoll kann über Eingabekomponenten, beispielsweise eine Maus, Tastatur, Pen, Touch-Display usw. geändert werden. In Antwort auf einen Befehl kann ein geändertes Konfigurationsprotokoll an das verteilte Datenbanksystem zur Speicherung und/oder Verteilung an wenigstens ein ausgewähltes bildgebendes Gerät gesendet werden. Die Änderungen an den Konfigurationsprotokollen können durch einen Applikationsingenieur, beispielsweise einen Physiker mit langjährigem und umfangreichem Kenntnisstand im Bereich von bildgebenden Geräten vorgenommen werden. Alternativ können Konfigurationsprotokolle basierend auf vorhergehenden Versionen neu erstellt werden. Der Befehl kann ein Betätigen und/oder Auswählen eines Buttons in der Anwendungssoftware beinhalten, was ein Senden von geänderten Konfigurationsprotokollen an das verteilte Datenbanksystem ausführt. Zudem ist im Sinne der vorliegenden Erfindung unter einem ausgewählten bildgebenden Gerät das bildgebende Gerät zu verstehen, für das das Konfigurationsprotokoll geändert wurde. Alternativ kann in einer Flotte von bildgebenden Geräten eine Vielzahl von bildgebenden Geräten die gleichen spezifischen Eigenschaften aufweisen, so dass alle bildgebenden Geräte der Gruppe das geänderte Konfigurationsprotokoll bereitgestellt bekommen und somit alle zu dem ausgewählten bildgebenden Gerät zugehörig sind. Damit können Änderungen an einem Konfigurationsprotokoll mit nur einem Befehl einheitlich für eine bestimmte Gruppe von Geräten ausgerollt werden.

In einem Offline-Betrieb können die Änderungen an den lokal gespeicherten Konfigurationsprotokollen in einer Ausführungsform ohne operative Kommunikation zwischen der Administratoreinheit und dem verteilten Datenbanksystem durchgeführt werden und als Antwort auf einen Befehl zum Senden an das verteilte Datenbanksystem vorbereitet gespeichert werden. Sobald eine operative Kommunikation zwischen der Administratoreinheit und dem verteilten Datenbanksystem (wieder) besteht, werden die geänderten Kommunikationsprotokolle automatisiert und/oder nach Empfang eines Sendebefehls an das verteilte Datenbanksystem gesendet. In einem Online-Betrieb können die Änderungen direkt über die operative Kommunikationsverbindung an das verteilte Datenbanksystem gesendet werden. Gemäß einer weiteren Ausführungsform ist die Administratoreinheit somit ausgebildet, die Konfigurationsprotokolle über die Benutzerschnittstelle lokal zu ändern und bei fehlender operativer Kommunikation mit dem verteilten Datenbanksystem diese lokal zu speichern und erst bei operativer Kommunikation automatisiert an das verteilte Datenbanksystem zu senden. In vorteilhafter Weise können Konfigurationsprotokolle geändert werden, wenn keine operative Kommunikation zwischen der Administratoreinheit und dem verteilten Datenbanksystem besteht. Die geänderten Konfigurationsprotokolle werden lokal in dem Speicher der Administratoreinheit gespeichert und bei einer operativen Kommunikation an das verteilte Datenbanksystem übertragen. Die Änderungen an den Konfigurationsprotokollen können unabhängig von dem Verwendungsort der bildgebenden Geräte und unabhängig einer bestehenden Verbindung zu den bildgebenden Geräten ausgeführt werden.

In vorteilhafter Weise wird durch die vorliegende Erfindung eine effiziente Bearbeitung umfassend ein Ändern und/oder Erstellen von Konfigurationsprotokollen für bildgebende Geräte über eine einzige Administratoreinheit ermöglicht. Somit wird der zeitliche Aufwand für die manuelle und separate Administration jedes einzelnen bildgebenden Gerätes in Summe reduziert. Zudem ist eine separate Administration vor Ort an den bildgebenden Geräten nicht mehr notwendig, da durch die auf dem verteilten Datenbanksystem zur Verfügung stehenden Konfigurationsprotokolle der bildgebenden Geräte gesammelt über eine einzige Administratoreinheit verwaltet/verarbeitet werden können. Hierdurch kann ein automatisierter Abgleich der Konfigurationsprotokolle über die gesamte Gruppe ermöglicht werden, was den Kommunikationsaufwand zwischen den Anwendern der verschiedenen bildgebenden Geräte reduziert und zugleich die notwendigen Hardwareressourcen für den Abgleich minimiert.

Weiterhin ist von Vorteil, dass notwendige Änderungen an den Konfigurationsprotokollen, beispielsweise an spezifischen Parametern für einen bestimmten Funktionsumfang eines bildgebenden Gerätes nicht übersehen und/oder vergessen werden können. Somit können Fehler während dem Ändern der Konfigurationsprotokolle vermieden werden, was die Zuverlässigkeit in der Ausführung der geänderten Konfigurationsprotokolle erhöht.

Zudem ist von Vorteil, dass die Änderungen nicht vor Ort bzw. lokal vorgenommen werden müssen bzw. in den Geräte-Host eingespielt werden müssen. Vielmehr können diese über einen Fernzugriff (Remote) von der Administratoreinheit angestoßen und über das verteilte Datenbanksystem in den Geräte-Host eingespielt (ausgerollt) werden. Dies kann während des Betriebes des bildgebenden Gerätes erfolgen, wodurch keine Betriebspausen des bildgebenden Gerätes notwendig sind.

Gemäß einer Ausführungsform umfasst das bildgebende Gerät ein Magnetresonanztomographie-Gerät (MRT-Gerät), ein Computertomografie-Gerät (CT-Gerät), ein Ultraschallgerät, ein Gerät zur Ausführung eines nuklearmedizinischen Verfahrens (z.B. SPECT, PET) oder ein Angiographiegerät. Das bildgebende Gerät weist spezifische Eigenschaften auf, die für das Verteilen der geänderte Konfigurationsprotokolle relevant sind. In vorteilhafter Weise können durch die vorteilhafte Ausgestaltung geänderte Konfigurationsprotokolle unter Berücksichtigung der spezifischen Eigenschaften verteilt werden. Weist z.B. eine erste Gruppe von Geräten eine erste Eigenschaft und eine zweite Gruppe von Geräten eine zweite Eigenschaft auf, so kann ein erstes Kommunikationsprotokoll für die erste und ein zweites für die zweite Gruppe ausgerollt werden. Entsprechend kann ein für einen Geräte-Host geändertes Konfigurationsprotokoll an weitere Geräte-Hosts von bildgebenden Geräten verteilt werden, welche die gleichen spezifischen Eigenschaften aufweisen. Zudem kann ein automatisiertes Verteilen (Ausrollen) der geänderten Konfigurationsprotokolle erfolgen. Die spezifischen Eigenschaften des bildgebenden Gerätes sind in entsprechenden Metadaten-Dateien hinterlegt.

Gemäß einer weiteren Ausführungsform sendet das bildgebende Gerät oder der zugeordnete Geräte-Host das Konfigurationsprotokoll gespiegelt an das verteilte Datenbanksystem. Im Sinne der vorliegenden Erfindung ist unter "gespiegelt" eine vollständige Kopie des Konfigurationsprotokolls, welches auf dem Geräte-Host ausgeführt wird, zu verstehen. In einer bevorzugten Ausführungsform sendet der Geräte-Host das Konfigurationsprotokoll automatisiert an das verteilte Datenbanksystem, sobald eine Kommunikationsverbindung zwischen dem Geräte-Host und dem verteilten Datenbanksystem besteht. In vorteilhafter Weise kann gewährleistet werden, dass das gespeicherte bzw. ausgeführte Konfigurationsprotokoll synchron und identisch auf zwei Einheiten lokal und zentral vorliegt.

Gemäß einer weiteren Ausführungsform umfasst das verteilte Datenbanksystem eine strukturierte Verzeichnisstruktur mit einem Unterverzeichnis für jede spezifische Software-Version der Geräte-Software, die auf dem Geräte-Host des jeweiligen bildgebenden Gerätes im Einsatz ist. Die strukturierte Verzeichnisstruktur kann in einer ersten Hierarchiestufe ein installiertes, erstes Unterverzeichnis für eine Gruppe von bildgebenden Geräten aufweisen. In einer Ausführungsform können auf dem verteilten Datenbanksystem weitere erste Unterverzeichnisse installiert sein, in denen andere Gruppen von bildgebenden Geräten zugeordnet sind. Ein erstes Unterverzeichnis der ersten Hierarchiestufe kann ein weiteres oder eine Vielzahl weiterer Unterverzeichnisse einer zweiten Hierarchiestufe aufweisen. Die Unterverzeichnisse der zweiten Hierarchiestufe können für jede spezifische Software-Version der Geräte-Software bereitgestellt werden. Somit können bildgebende Geräte mit der gleichen Software-Version in dem gleichen Unterverzeichnis gruppiert werden. Diese strukturiere Verzeichnisstruktur ermöglicht es, in dem gleichen verteilten Datenbanksystem verschiedene Geräte-Hosts zu verwalten. Somit ist eine geordnete Struktur der gespeicherten Daten trotz eines Mischbetriebes unterschiedlicher Software-Versionen gegeben. Durch das Einhalten der strukturierte Verzeichnisstruktur und der Verwendung der Metadaten-Dateien in jeder Software-Version, ist es ferner möglich, auf Unterverzeichnisse älterer Software-Versionen zu zugreifen und bei Bedarf (z.B. bei einer Recovery) einen automatisierten Import zu initialisieren.

Gemäß einer weiteren Ausführungsform ist für jede spezifische Software-Version der Geräte-Software eine zentrale Datenbank in einem spezifischen Unterverzeichnis des verteilten Datenbanksystems entsprechend ihrer Software-Version eingerichtet. Die Separierung der Datenbanken auf spezifische Unterverzeichnisse entsprechend ihrer kompatiblen Software-Version ist dahingehend vorteilhaft, dass Probleme bei nicht vorhandener Vorwärts-Kompatibilität vermieden werden können. Zudem sind über die strukturierte Verzeichnisstruktur ein Zugriff auf und ein automatisierter Import von Konfigurationsprotokollen älterer Software-Versionen möglich. Dies ist gerade während eines Upgrades einer Geräte-Software von Vorteil, insbesondere dann, wenn der Geräte-Host der erste in der Gruppe ist, auf dem eine neue Software-Version installiert ist und zum Erstellen einer neuen zentralen Datenbank auf Quellen der Vorgängerversion zugegriffen werden muss. Wird bei einem Zugriff auf ein Unterverzeichnis einer früheren Version eine entsprechende zentrale Datenbank gefunden, kann diese zentrale Datenbank zur Datenübernahme verwendet und automatisiert konvertiert werden. Über diesen Mechanismus ist ein manueller Eingriff durch, beispielsweise einen Applikationsingenieur vor Ort nicht notwendig und eine fehlerfreie Datenübernahme ist gewährleistet. Eine Datenübernahme aus einer Vorgänger-Version ist automatisiert möglich. Zudem kann die Softwareentwicklung unabhängiger gestaltet werden, da Inkompatibilitäten der zentralen Datenbanken zueinander, insbesondere zwischen den verschiedenen Software-Versionen möglich sind.

Gemäß einer weiteren Ausführungsform ist der Name des Unterverzeichnisses aus der Major-Version der ausgeführten Geräte-Software und einem Iterationswert bestimmt. Die Major-Version stellt die Version der Software dar, die der ursprünglich ausgelieferten Version ohne ein eingespieltes Update entspricht. Diesbezüglich wurden noch keine Änderungen/Anpassungen an den Konfigurationsprotokollen vorgenommen. Der Iterationswert wird erhöht, vorzugsweise automatisiert erhöht, wenn innerhalb der Major-Version eine Inkompatibilität der Software (geändertes Konfigurationsprotokoll) zur Datenbank vorliegt.

Gemäß einer weiteren Ausführungsform ist in jedem softwareversionsspezifischen Unterverzeichnis eine Metadaten-Datei eingerichtet. Die Metadaten-Datei umfasst entsprechende Informationen über die auf dem Host-Gerät installierte Software-Version der Gerätesoftware. Insbesondere umfasst die Metadaten-Datei detaillierte Information bezüglich dem zu der Software-Version zugehörigen Konfigurationsprotokoll. Die Metadaten-Datei kann automatisch durchsucht und ausgewertet werden, um so eine Zuordnung zwischen dem bildgebenden Gerät (Geräte-Host) und dem Unterverzeichnis zu gewährleisten.

Gemäß einer weiteren Ausführungsform werden geänderte Konfigurationsprotokolle automatisiert an die Geräte-Hosts der bildgebenden Geräte verteilt. Durch die vorliegende Erfindung kann ein automatisiertes Verteilen erfolgen, da vor dem Verteilen die neue Version des Konfigurationsprotokolls automatisch auf die spezifischen Eigenschaften des neuen Geräte-Hosts konvertiert wurde, ohne dass ein Applikationsingenieur den manuell den Konvertierungsvorgang ausführen muss. Der Applikationsingenieur bekommt beispielsweise angezeigt wo Konvertierungsunterschiede vorliegen, welche überprüft und übernommen werden müssen. Hierdurch ist das automatisierte Verteilen der Konfigurationsprotokolle an die bildgebenden Geräte sowohl zeit- als auch ressourcensparender und somit effizienter.

Gemäß einer weiteren Ausführungsform werden die geänderten Konfigurationsprotokolle instantan an die Geräte-Hosts der bildgebenden Geräte verteilt. Dies ermöglich eine schnelle und effiziente Aktualisierung der geänderten Konfigurationsprotokolle.

Gemäß einer weiteren Ausführungsform werden die Konfigurationsprotokolle nach einem vordefinierten Kriterium automatisiert verteilt. Das Kriterium kann eine Freigabezeit, eine Dauer oder vorkonfigurierbare Bedingungen umfassen. Weiterhin kann das Kriterium festlegen, dass die Konfigurationsprotokolle erst verteilt werden, wenn für jedes bildgebende Gerät einer Gruppe ein entsprechendes geändertes Konfigurationsprotokoll bereitsteht. Somit wird gewährleistet das jedes bildgebende Gerät einer Gruppe den gleichen Stand des geänderten Konfigurationsprotokolls aufweist. Dies ist von Vorteil, wenn ein Anwender eine Vielzahl an bildgebenden Geräten mit der gleichen Optimierung zu einem bestimmten Zeitpunkt betreiben möchte.

Gemäß einer weiteren Ausführungsform ist ein für das bildgebendes Gerät geänderte Konfigurationsprotokoll nur für dieses bildgebende Gerät sichtbar und/oder zugreifbar. Somit kann auf einem bildgebenden Gerät, welches nicht kompatibel ist, keine geändertes Konfigurationsprotokoll eingespielt werden. Über die strukturierte Verzeichnisstruktur können inkompatible von den zueinander kompatiblen bildgebenden Geräten getrennt werden. Damit kann die Sicherheit beim Betrieb der Geräte verbessert werden. Insbesondere kann sichergestellt werden, dass das Gerät nur ihr "eigenes" Konfigurationsprotokoll und keine "fremden" einsehen kann.

Gemäß einer weiteren Ausführungsform sind die in dem verteilten Datenbanksystem abgespeicherten Daten zugriffsgeschützt abgespeichert. Durch den Zugriffschutz kann keine Änderungen an den Konfigurationsprotokollen durch nicht autorisierte Anwender vorgenommen werden. Der Zugriffsschutz kann beispielsweise über eine Eingabe eines Kennwortes softwaretechnisch in der Anwendungssoftware ausgebildet sein. Weiterhin kann der Zugriffsschutz hardwaretechnisch über eine Freigabe der Administratoreinheit über eine Kennworteingabe an der Administratoreinheit, über einen Dongle usw. erfolgen. Ohne eine entsprechende Zugriffsberechtigung wird der Lese- und/oder Schreibzugriff auf die Konfigurationsprotokolle verwehrt.

Über den Zugriffsschutz kann ebenfalls ein nichtberechtigtes Geräte-Host für einen Zugriff auf Konfigurationsprotokolle ausgeschlossen werden.

Gemäß einer weiteren Ausführungsform ist die Administratoreinheit ausgebildet, über den Prozessor eine Validierungsalgorithmus zur Validierung der geänderten Konfigurationsprotokolle auszuführen. Der Validierungsalgorithmus kann zentral auf der Administratoreinheit nach einem Ändern eines Konfigurationsprotokolls ausgeführt werden. Alternativ kann der Validierungsalgorithmus im Hintergrund aktiv ausgeführt werden und eine on-the-fly Validierung der Änderungen an dem Konfigurationsprotokoll durchführen. Unzulässige Änderungen und/oder Eingaben können für eine anschließende Korrektur gekennzeichnet werden, so dass nur zulässige Eingaben möglich sind. Die Änderungen können mit Bezug auf die spezifischen Eigenschaften des bildgebenden Gerätes validiert werden. Somit kann sichergestellt werden, dass das geänderten Konfigurationsprotokoll auf dem Geräte-Host des zugeordneten bildgebenden Gerätes ausführbar bzw. ladbar ist und für das bildgebende Gerät anwendbar ist.

In einer Ausführungsform kann durch den Validierungsalgorithmus eine entsprechende Konvertierung der nicht validen Änderungen vorgeschlagen werden. Weiterhin kann durch den Validierungsalgorithmus eine entsprechende Konvertierung der nicht validen Änderungen durchgeführt werden.

Gemäß einer weiteren Ausführungsform sind die geänderten Konfigurationsprotokolle gegenüber den spezifischen Eigenschaften des bildgebenden Gerätes validiert. In vorteilhafter Weise sind die geänderten Konfigurationsprotokolle funktional validiert und können an das zugehörige bildgebende Gerät verteilt/ausgerollt werden.

Gemäß einem zweiten Aspekt betrifft die Erfindung ein computerimplementiertes Verfahren zur Administration von bildgebenden Geräten. Das computerimplementierte Verfahren umfasst die folgenden Schritte:
- Senden eines auf einem Geräte-Host eines bildgebenden Gerätes installierten Konfigurationsprotokolls an ein verteiltes Datenbanksystem;
- Empfangen des gesendeten Konfigurationsprotokolls und Speichern des empfangenen Konfigurationsprotokolls für den Geräte-Host durch das verteilte Datenbanksystem;
- Bereitstellen des gespeicherten Konfigurationsprotokolls für eine Administratoreinheit zum Erzeugen eines geänderten Konfigurationsprotokolls mittels einer Anwendungssoftware, die auf der Administratoreinheit ausgeführt wird; und
- Empfangen eines Befehls über die Anwendungssoftware und Senden des geänderten Konfigurationsprotokolls in Antwort auf den empfangenen Befehl an das verteilte Datenbanksystem zur Speicherung und/oder Verteilung an wenigstens ein ausgewähltes bildgebendes Gerät.

Die vorstehend beschriebene, erfindungsgemäße Ausführungsform des Verfahrens gemäß dem zweiten Aspekt der Erfindung kann auch als ein Computerprogramm ausgebildet sein, wobei ein Computer zur Durchführung des oben beschriebenen, erfindungsgemäßen Verfahrens veranlasst wird, wenn das Computerprogramm auf einem Computer bzw. auf einem Prozessor des Computers ausgeführt wird. Das Computerprogramm kann auf ein Signal hin per Download bereitgestellt oder in einer Speichereinheit des Computers oder der Administratoreinheit mit darin enthaltenem computerlesbarem Programmcode gespeichert werden, um die Administratoreinheit, einen Server zur Ausführung von Anweisungen gemäß dem oben genannten Verfahren zu veranlassen. Dabei kann das Computerprogramm auch auf einem maschinenlesbaren Speichermedium gespeichert sein. Eine alternative Aufgabenlösung sieht ein Speichermedium vor, das zur Speicherung des vorstehend beschriebenen, computer-implementierten Verfahrens bestimmt ist und von einem Computer oder Prozessor lesbar ist.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird dabei der Fachmann auch Einzelaspekte als Verbesserung oder Ergänzungen zu der jeweiligen Grundform der vorliegenden Erfindung hinzufügen.

Die vorliegende Erfindung wird nachfolgend anhand der in den schematischen Figuren der Zeichnungen angegebenen Ausführungsbeispiele näher erläutert. Es zeigen dabei:
- Fig. 1: eine schematische Darstellung einer Ausführungsform eines Systems zur Administration von bildgebenden Geräten;
- Fig. 2: eine schematische Darstellung einer weiteren Ausführungsform eines Systems zur Administration von bildgebenden Geräten; und
- Fig. 3: ein Ablaufdiagramm gemäß einer Ausführungsform des erfindungsgemäßen computerimplementierten Verfahrens.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche, und gleich wirkende Elemente, Merkmale und Komponenten - sofern nichts anderes ausgeführt ist - jeweils mit denselben Bezugszeichen zu versehen.

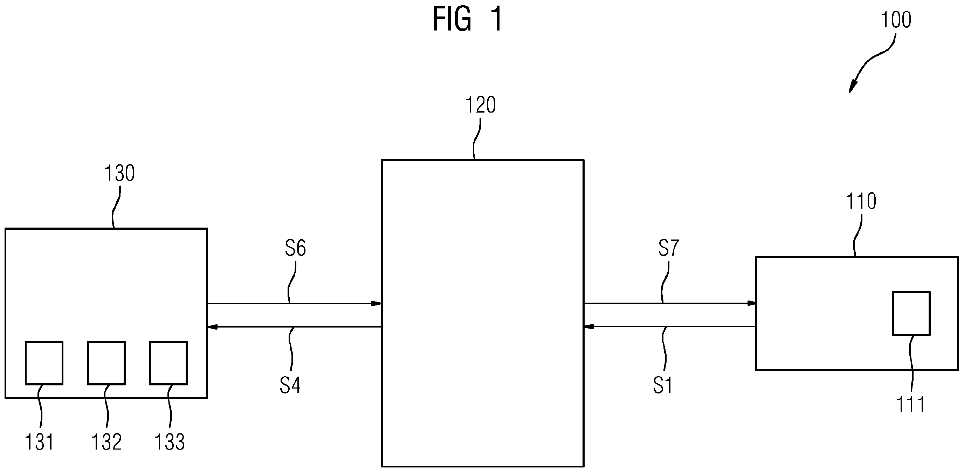

Fig. 1 zeigt eine schematische Darstellung einer Ausführungsform eines Systems zur Administration von bildgebenden Geräten. Der Begriff "Administration" umfasst ein Ansteuern zum Betreiben des Gerätes. Administration ist somit im Sinne eines technischen Schrittes zu verstehen, der den Betrieb des Gerätes umfassen kann. Administrieren bezieht sich somit auf die Bereitstellung der technischen Grundlagen (insbesondere mit den Konfigurationsprotokollen, Kompatibilitätsprüfungen etc.) für die Ausführung der Software auf den Hosts für die Geräte.

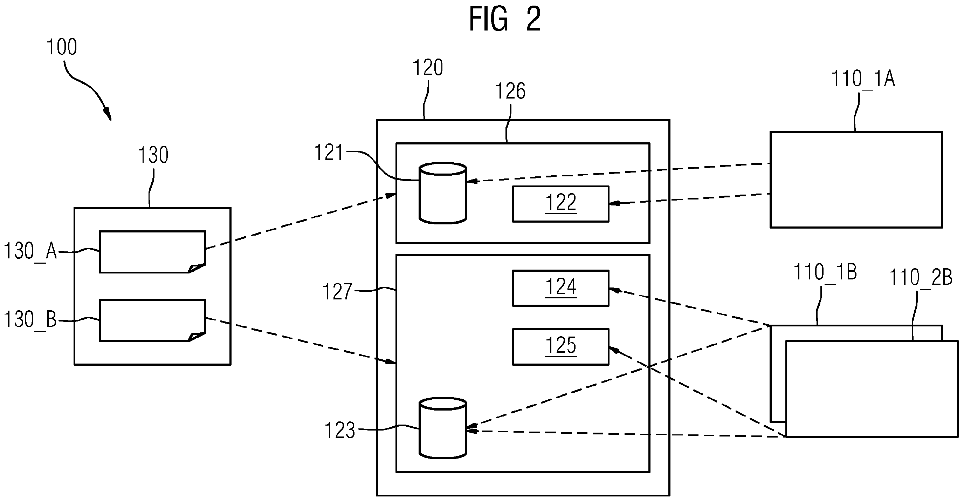

Das System 100 umfasst mindestens ein bildgebendes Gerät 110 einer Gruppe oder Flotte, die administriert werden muss, z.B. in einer Klinik. Die Vielzahl der bildgebenden Geräte 110 können einer Gruppe an bildgebenden Geräten 110 zugeordnet werden und über das System 100 administriert werden. Das bildgebende Gerät 110 kann als ein Magnetresonanztomographie-Gerät (MRT-Gerät) oder als ein Computertomografie-Gerät (CT-Gerät) ausgebildet sein. Ferner umfasst das bildgebende Gerät 110 einen Geräte-Host 111. Das Geräte-Host 111 ist als eine Recheneinheit mit einem flüchtigen und/oder nichtflüchtigen Speicher, wenigstens einem Prozessor und Kommunikationsschnittstellen zur Kommunikation mit dem bildgebenden Gerät 110 und zur Kommunikation mit einem Anwender des bildgebenden Gerätes 110 ausgebildet. Ferner kann das Geräte-Host 111 Kommunikationsschnitten zur Kommunikation mit weiteren Geräten, beispielsweise für die Bedienung aufweisen. Die Kommunikationsschnittstellen können für eine kabelgebundene und/oder kabellose Kommunikation ausgebildet sein. Insbesondere kann der Geräte-Host 111 als ein Computer, ein Laptop, eine Workstation etc. ausgebildet sein. Der Geräte-Host 111 ist konfiguriert eine Geräte-Software, ein sogenanntes Binary, zum Bedienen und Ansteuern des bildgebenden Gerätes 110 zu speichern und auszuführen. Über das Geräte-Host 111 werden die Bildaufnahmen des bildgebenden Gerätes 110 gesteuert und/oder ausgewertet bzw. zur Auswertung bereitgestellt. Auf dem Geräte-Host 111 werden die Geräte-Software und das Konfigurationsprotokoll 122, 124, 125 gemäß der Fig. 2 ausführbar gespeichert. Die Geräte-Software (Binary) dient dem Ansteuern und Bedienen des bildgebenden Gerätes 110. Das Konfigurationsprotokoll 122, 124, 125 umfasst die Parametereinstellungen des bildgebenden Gerätes 110 entsprechend den spezifischen Eigenschaften des bildgebenden Gerätes 110. Über das Konfigurationsprotokoll 122, 124, 125 können entsprechende Parameter verändert werden, um beispielsweise die Bildaufnahmen der bildgebenden Geräte 110 entsprechend den Anforderungen zu ändern.

Jedes bildgebende Geräte 110 einer Gruppe weist spezifische Eigenschaften auf, die beispielsweise über Lizenzen freigeschaltet sind. Die spezifischen Eigenschaften können an einen zentralen Speicher bereitgestellt werden, sobald ein bildgebende Gerät 110 einer Gruppe zugeordnet ist. Das Zuordnen einer Gruppe kann das technische Einbinden bzw. den Aufbau einer Kommunikation umfassen. Als zentralen Speicher weist das System 100 ein verteiltes Datenbanksystem 120 auf. Das verteilte Datenbanksystem 120 weist eine Kommunikationsverbindung zu jedem Geräte-Host 111 des bildgebenden Gerätes 110 einer Gruppe auf. Über die Kommunikationsverbindung empfängt das verteilte Datenbanksystem 120 das Konfigurationsprotokoll 122, 124, 125 von jedem Geräte-Host 111 des der Gruppe zugeordneten bildgebenden Gerätes 110. Die jeweiligen Konfigurationsprotokolle 122, 124, 125 werden gespiegelt in dem verteilten Datenbanksystem 120 gespeichert. Das verteilte Datenbanksystem 120 kann aus einer Vielzahl von physisch getrennten Datenbanken aufgebaut sein, welche unabhängig zueinander arbeiten und wie eine einzige logische Datenbank verwaltbar sind. Auf die verteilte Datenbank 120 kann zentral zugegriffen werden. Das verteilte Datenbanksystem 120 kann zugriffsgeschützt ausgeführt sein, bei der nicht autorisierten Anwendern kein Lese- und/oder Schreibschutz gewährt wird. Weiterhin weist das System 100 eine Administratoreinheit 130 auf. Die Administratoreinheit 130 kann als ein Computer oder Handheld, beispielsweise ein Laptop, Tablet, Smartphone usw. ausgebildet sein. Die Administratoreinheit 130 umfasst einen flüchtigen und/oder nichtflüchten Speicher 131, beispielsweise RAM, ROM, Flash, Festplattenspeicher zum lokalen Speichern einer Firmware, eines Betriebssystems, einer Anwendungssoftware und/oder von Anwendungsdaten, beispielsweise den Konfigurationsprotokollen 122, 124, 125. Weiterhin umfasst die Administratoreinheit 130 eine Benutzerschnittstelle 132, beispielsweise ein Display, auf der eine Interaktion zwischen einem Anwender und der Administratoreinheit erfolgen kann. Eine Interaktion kann beispielsweise ein Anzeigen von Konfigurationsprotokollen 122, 124, 125 beispielsweise zur Analyse und/oder eine Eingabe von Änderungen an den Konfigurationsprotokollen 122, 124, 125 umfassen. Ferner weist die Administratoreinheit Kommunikationsschnittstellen 132 zur operativen Kommunikation eines Prozessors 133 mit dem verteilten Datenbanksystem 120 auf. Die Kommunikationsschnittstellen 132 können als kabelgebundene Kommunikationsschnittstellen, beispielsweise Ethernet oder als kabellose Kommunikationsschnittstellen, beispielsweise WLAN ausgebildet sein. In dem Speicher 131 der Administratoreinheit 130 kann eine Anwendungssoftware zum Verwalten und/oder Bearbeiten von lokal gespeicherten Konfigurationsprotokollen 122, 124, 125 gespeichert sein, die durch einen Prozessor 133 der Administratoreinheit 130 ausgeführt wird. In einer Ausführungsform kann die Administratoreinheit 130 eine Vielzahl an Prozessoren 133 oder Prozessoren mit einer Vielzahl an Prozessorkernen aufweisen. In einer alternativen Ausführungsform kann die Administratoreinheit 130 eine Anwendungssoftware zum Zugriff auf webbasierte Inhalte gespeichert bereitstellen, mittels der webbasiert Konfigurationsprotokolle 122, 124, 125 verwaltet und/oder bearbeitet werden können.

Über die auf der Administratoreinheit 130 gespeicherte und ausführbare Anwendungssoftware kann das Konfigurationsprotokoll 122, 124, 125 jedes mit dem System 100 verbundenen bildgebenden Gerätes 110, einschließlich der spezifischen Eigenschaften abgerufen und lokal gespeichert werden. Über die Administratoreinheit 130 kann, beispielsweise ein Applikationsingenieur entsprechend gewünschte Anforderungen an die bildgebenden Geräte 110 umsetzen und die Konfigurationsprotokolle 122, 124, 125 entsprechend den Anforderungen ändern. In vorteilhafter Weise ist eine operative Kommunikation während der Bearbeitung der Konfigurationsprotokolle 122, 124, 125 nicht notwendig. Die Konfigurationsprotokolle 122, 124, 125 können somit offline geändert werden. In Antwort auf einen über die Anwendungssoftware empfangenen Befehl werden die geänderten Konfigurationsprotokolle 130_A, 130_B bei einer bestehenden operativen Kommunikation auf das verteilte Datenbanksystem 120 übertragen und gespeichert. Vorhandene Konfigurationsprotokolle 122, 124, 125 insbesondere ältere Versionen werden nicht durch die geänderten Konfigurationsprotokolle 130_A, 130_B überschrieben und/oder ersetzt. Durch das verteilte Datenbanksystem 120 wird eine Inkompatibilität zwischen den neuen Konfigurationsprotokollen 122, 124, 125 und der zentralen Datenbank 121, 123 (vgl. Fig. 2) festgestellt. In der strukturierten Verzeichnisstruktur der verteilten Datenbank 120 wird entsprechend der neuen Version ein neues Unterverzeichnis angelegt. Die Bezeichnung des neuen Unterverzeichnisses wird aus der Major-Version der ursprünglich (werkseitig) auf dem Geräte-Host 111 eingespielten Version und einem Iteratorwert erstellt. Der Iteratorwert wird mit jeder neuen Version im Wert erhöht. Über das verteilte Datenbanksystem 120 können die geänderten Konfigurationsprotokolle 130_A, 130_B an die Geräte-Hosts 111 der bildgebenden Geräte 110 automatisiert verteilt werden. Ein vor Ort Export der Konfigurationsprotokolle 122, 124, 125, sowie das Ändern und manuelle wieder Importieren des geänderten Konfigurationsprotokoll 130_A, 130_B ist in vorteilhafter Weise nicht notwendig. Zudem kann das automatisierte Verteilen der geänderten Konfigurationsprotokolle 122, 124, 125 während des Betriebs der bildgebenden Geräte 110 erfolgen. Weiterhin vorteilhaft ist, dass über eine Administratoreinheit 130 die Konfigurationsprotokolle 130_A, 130_B einer Vielzahl, beispielsweise 100 bildgebende Geräte bearbeitet bzw. geändert und die Änderungen automatisiert verteilt werden können.

Fig. 2 zeigt eine schematische Darstellung einer Ausführungsform eines Systems 100 zur Administration von bildgebenden Geräten 110. Das System 100 der in Fig. 2 dargestellten Ausführungsform umfasst drei bildgebende Geräte 110. Insbesondere umfasst das System 100 ein bildgebendes Gerät 110_1A und zwei weitere bildgebende Geräte 110_1B, 110_2B. Das bildgebende Gerät 110_1A weist beispielsweise eine Geräte-Software mit einer Software-Version "VA20A" auf. Die zwei bildgebenden Geräte 110_1B, 110_2B weisen beispielsweise eine Geräte-Software mit einer Software-Version "VA30A" auf. Hierbei stellen "VA20A", "VA30B" unterschiedliche Software-Versionen oder Software-Stände der Geräte-Software dar. Die beispielshafte Benennung der Software-Version der Geräte-Software stellt hierbei keine einschränkende Begrenzung dar. Die Benennung wurde nur zur Erläuterung und Veranschaulichung gewählt. Vielmehr können auch andere Bezeichnungen gewählt werden. Die bildgebenden Geräte 110 der in der Fig. 2 dargestellten Ausführungsform können einer Gruppe zugeordnet sein. Für jede Software-Version der Geräte-Software eines bildgebenden Gerätes 110_1A, 110_1B, 110_2B wird in der verteilten Datenbank ein spezifischer Unterorder eingerichtet, in der das entsprechend zugehörige Konfigurationsfile 122, 124, 125 gespiegelt gespeichert ist. Zudem wird für jede Software-Version der Geräte-Software des bildgebenden Gerätes 110_1A, 110_1B, 110_2B in dem spezifischen Ordner in der verteilten Datenbank eine zentrale Datenbank 121, 123 eingerichtet. In der folgenden Auflistung ist eine beispielhafte strukturierte Verzeichnisstruktur der verteilten Datenbank 120 dargestellt.

### Strukturierte Verzeichnisstruktur der verteilten Datenbank 120

```
 \\server\gruppe\VA20A_1\gruppe@@0.exdb1
 \\server\gruppe\VA20A_1\Gerät-01\GerätSummary.State
 \\server\gruppe\VA20A-1\Gerät-02\GerätSummary.state
 \\server\gruppe\VA30A_1\gruppe@@0.exdbl
 \\server\gruppe\VA30A_1\Gerät-01\GerätSummary.State
 \\server\gruppe\VA30A-1\Gerät-02\GerätSummary.state
```

In dem dargestellten Pfad der strukturierten Verzeichnisstruktur der verteilten Datenbank 120 wird durch "Server" die verteilte Datenbank 120 bezeichnet. Der Pfad ist bei allen bildgebenden Geräten 100 identisch. Die bildgebenden Geräte 100 sind einer Gruppe an bildgebenden Geräten 100 zugeordnet. Die Bezeichnung "gruppe" stellt nur eine beispielhafte Bezeichnung für eine Gruppe dar, welche zugeordnete bildgebende Geräte 100 umfasst. Für jede Software-Version der Geräte-Software VA20A_1, VA30A_1 ist ein Unterverzeichnis eingerichtet. In jedem der Unterverzeichnisse ist eine zentrale Datenbank ".exdb1" und das Konfigurationsprotokoll 122, 124, 125 der entsprechenden bildgebenden Geräte 110_1A, 110_1B, 110_2B gespeichert. Beispielsweise gibt es in der dargestellten Gruppe bildgebende Geräte 110_1A, 110_1B, 110_2B, auf denen die Software-Version "VA20A_1" (bildgebendes Gerät 110_1A) und die Software "VA30A_1" (bildgebende Geräte 110_1B, 110_2B) eingerichtet sind. Die bildgebenden Geräte 110_1A, 110_1B, 110_2B werden entsprechend der Software-Version gruppiert, (VA20A, VA30A), da diese unterschiedliche zentrale Datenbanken 121, 123 aufweisen und somit unterschiedlich behandelt werden. Entsprechend der Software-Version der Geräte-Software weisen die bildgebenden Geräte 110 ein Konfigurationsprotokoll 122, 124, 125 auf. Die Bezeichnungen "VA20A" und "VA20B" stellen hierbei den eingerichteten Softwarestand für jedes der bildgebenden Geräte 110_1A, 110_1B, 110_2B dar. Die Bezeichnung "_1" stellt hierbei den fortlaufenden Iteratorwert für vorgenommene Änderungen an dem Konfigurationsprotokoll 122, 124, 125 dar. Der Iteratorwert erhöht sich, wenn das verteilte Datenbanksystem 120 ein neues Konfigurationsprotokoll 122, 124, 125 empfängt und speichert, welches eine Inkompatibilität zu der zentralen Datenbank 121, 123 in dem zugehörigen Unterverzeichnis aufweist. Diesbezüglich wird ein neues Unterverzeichnis mit einer positiv iterierten Unterverzeichnisbezeichnung angelegt und entsprechende Softwareteile der vorherigen Version, welche nicht geändert sind, können für die neue Software-Version übernommen werden. Somit kann ein automatisierter Konvertierungsvorgang erfolgen. Die Bezeichnungen "Gerät-01" und "Gerät-02" stellen eine exemplarische Bezeichnung der bildgebenden Geräte 110 dar und kann frei gewählt werden. Die Bezeichnung "-02" stellt eine exemplarische fortlaufende Bezeichnung der bildgebenden Geräte 110 einer Gruppe an bildgebenden Geräte 110 entsprechend einer Software-Version dar. In diesem Unterverzeichnis wird die Metadaten-Datei für das bildgebende Geräte 110 eingerichtet. Folgend ist eine Pseudocode einer Metadaten-Datei dargestellt. Dieser Pseudocode stellt keine Beschränkung des Inhaltes der Metadaten-Datei dar, sondern soll nur beispielhaft deren Funktion beschreiben.

### Metadaten-Datei

```
 {
  "Major-Version": "VA30A",
  "Protocoll": "123456789",
  "AddIn": "NXXXXXX",
  "Edf": "1",
  "Sequence": "1"
 }
```

Die Metadaten-Datei umfasst beispielsweise die aktuell einrichte Software-Version der Geräte-Software, die Version des Konfigurationsprotokoll 122, 124, 125 welches durch das Geräte-Host 111 des bildgebenden Gerätes 110 mit der Software-Version der Geräte-Software ausgeführt wird. Weiterhin kann die Metadaten-Datei die IP-Adresse umfassen und weitere Parameter umfassen. In der Metadaten-Datei ist die Softwarekompatibilität mit der zentralen Datenbank 120 hinterlegt.

Das in der Fig. 2 dargestellte verteilte Datenbanksystem 120 weist für jede auf dem Geräte-Host 111 der bildgebenden Geräte 110 installierte Software-Version der Geräte-Software ein entsprechendes Unterverzeichnis 126, 127 in der strukturierten Verzeichnisstruktur auf. Das Unterverzeichnis 126 speichert beispielsweise das Konfigurationsprotokoll 122 der Software-Version "VA20A". In dem Unterverzeichnis 127 ist beispielsweise das Konfigurationsprotokoll 124, 125 die Software-Version "VA30A" gespeichert. Die Konfigurationsprotokolle 122, 124, 125 werden von den Geräte-Hosts 111 der bildgebenden Geräte 110 gespiegelt, sobald diese in der Gruppe aufgenommen sind. Die Konfigurationsprotokolle 122, 124, 125 können beispielsweise das Datenformat ".xml", ".dat" aufweisen. Dies stellt keine Beschränkung dar. Es ist denkbar das andere Datenformate verwendet werden, welche ebenfalls durch ein Anwendungsprogramm gelesen und verarbeitet werden können. Zudem umfasst das Unterverzeichnis 126 alle bildgebenden Geräte 110 auf deren Geräte-Host die Geräte-Software "VA20A" ausgeführt wird.

Folgend ist eine beispielhafte Ausgestaltung der strukturierten Verzeichnisstruktur des verteilten Datenbanksystems 120 dargestellt.
◊ Server
∘ Gruppe
▪ VA20A-01
▪ VA20A-02
▪ VA20A-03
▪ VA30A-01
▪ VA30A-02

Für jede Software-Version der Geräte-Software einen bildgebenden Gerätes 110 wird ein versionsspezifisches Unterverzeichnis 126, 127 eingerichtet, in der eine Metadaten-Datei mit den entsprechenden Metadaten gespeichert ist. Die Metadaten-Datei kann automatisiert durchsucht und ausgewertet werden, um so eine Zuordnung von bildgebendem Gerät 110 und Unterverzeichnis 126, 127 zu gewährleisten.

Weiterhin ist in der Fig. 2 die Administratoreinheit 130 dargestellt. Die Administratoreinheit 130 bekommt über das verteilte Datenbanksystem 120 die entsprechenden Konfigurationsprotokolle 122, 124, 125 bereitgestellt. Die Informationen der Konfigurationsprotokolle 122, 124, 125 werden in die Anwendungssoftware geladen. Die Konfigurationsprotokolle 122, 124, 125 können über eine Anwendungssoftware eingesehen und bearbeitet bzw. geändert werden.

Die geänderten Konfigurationsprotokolle 130_A, 130_B werden als Antwort auf einen Befehl in der Anwendungssoftware an das verteilte Datenbanksystem 120 gesendet. Wird ein Unterschied zwischen den geänderten Konfigurationsprotokollen 130_A, 130_B und den auf den Geräte-Hosts 111 ausgeführten Konfigurationsprotokollen 122, 124, 125 erkannt, wird ein neues Unterverzeichnis 126, 127 für die neue Version der Konfigurationsprotokolle 122, 124, 125 angelegt. Der Unterschied wird durch einen Abgleich mit der zentralen Datenbank 121, 123 ermittelt. Nicht geänderte Bestandteile der vorhergehenden Version werden bereits in der Administratoreinheit 130 hinzugefügt und sind somit in dem neuen Unterverzeichnis 126, 127 automatisiert übernommen. Somit kann eine automatisierte Konvertierung erfolgen und ein manuelles Eingreifen ist nicht mehr notwendig. Beispielsweise kann auf der Administratoreinheit 130 eine neues Konfigurationsprotokoll 122, 124, 125 entwickelt werden. Dieses kann in ein bestehendes auf der Administratoreinheit 130 gespeichertes Konfigurationsprotokoll 122, 124, 125 eingefügt werden. Mit der Konvertierungsfunktion in der Anwendersoftware der Administratoreinheit 130 kann das neue Konfigurationsprotokoll 122, 124, 125 konvertiert werden. Nach der Konvertierung kann als Antwort auf einen Befehl können das oder die geänderten Konfigurationsprotokolle 122, 124, 125 an das zentrale Datenbanksystem 120 zur Verteilung auf die Geräte-Hosts 111 der bildgebenden Geräte 110 gesendet werden. Ferner kann über die Administratoreinheit 130 und die gespeicherten Konfigurationsprotokolle 122, 124, 125 eine automatisierte Validierung der vorgenommenen Änderung erfolgen.

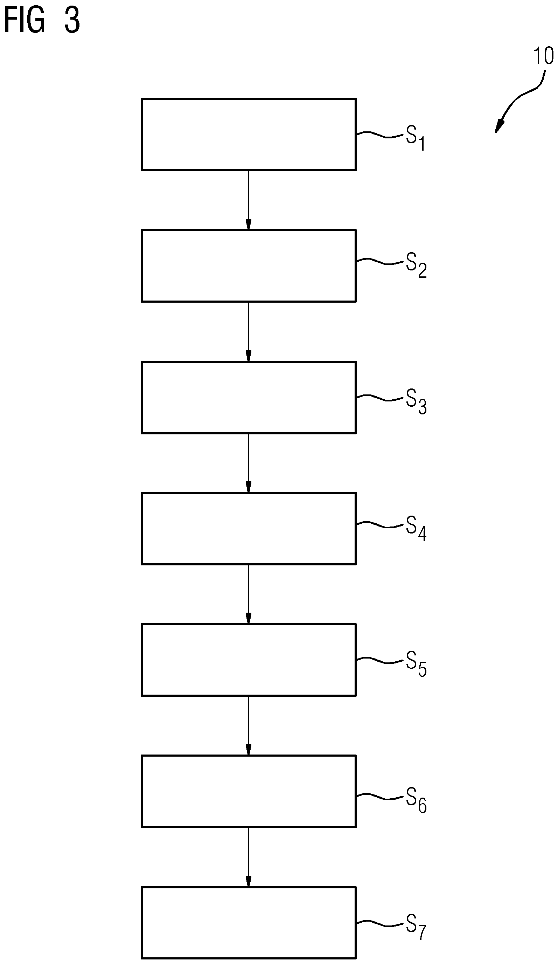

Fig. 3 zeigt ein Ablaufdiagramm gemäß einer Ausführungsform des erfindungsgemäßen computerimplementierten Verfahrens 10. Das Verfahren 10 umfasst bei der dargestellten Ausführungsform mehrere Schritte. In einem ersten Schritt S1 wird ein auf einem Geräte-Host 111 eines bildgebenden Gerätes 110 installiertes Konfigurationsprotokoll 122, 124, 125 an ein verteiltes Datenbanksystem 120 gesendet. Das installierte Konfigurationsprotokoll 122, 124, 125 entspricht der aktuellen Version, welche für den Betrieb des bildgebenden Gerätes 110 verwendet wird. Die installierte Konfiguration wird auf das verteilte Datenbanksystem 120 gespiegelt. In einem weiteren Schritt S2 wird das gesendete Konfigurationsprotokoll 122, 124, 125 empfangen, insbesondere durch das verteilte Datenbanksystem 120 empfangen. In einem folgenden Schritt S3 wird das empfangene Konfigurationsprotokoll 122, 124, 125 für den Geräte-Host 111 durch das verteilte Datenbanksystem 120 gespeichert. In dem Schritt S4 wird das gespeicherte Konfigurationsprotokoll 122, 124, 125 für eine Administratoreinheit 130 zum Erzeugen eines geänderten Konfigurationsprotokolls 130_A, 130_B mittels einer Anwendungssoftware, die auf der Administratoreinheit 130 ausgeführt wird, bereitgestellt. In einem weiteren Schritt S5 wird ein Befehl über die Anwendungssoftware empfangen. Der Befehl kann durch einen Button in der Anwendungssoftware oder durch eine Trigger-Signal, ausgelöst durch die Administratoreinheit 130 bereitgestellt werden. In einem weiteren Schritt S6 erfolgt das Senden des geänderten Konfigurationsprotokolls 130_A, 130_B in Antwort auf den empfangenen Befehl an das Verteilte Datenbanksystem 120 zur Speicherung in dem verteilten Datenbanksystem. In einem weiteren Schritt S7 wird das geänderte Konfigurationsprotokoll 130_A, 130_B an wenigstens ein ausgewähltes bildgebendes Gerät 110 verteilt, vorzugsweise automatisiert verteilt.

## Patentansprüche

1. System (100) zur Administration von bildgebenden Geräten (110), umfassend:
mindestens ein bildgebendes Gerät (110) mit einem Geräte-Host (111), wobei auf dem Geräte-Host (111) eine Geräte-Software in einer spezifischen Software-Version und ein Konfigurationsprotokoll (122, 124, 125) ausführbar (111) gespeichert sind;
ein verteiltes Datenbanksystem (120), das konfiguriert ist, Konfigurationsprotokolle (122, 124, 125) von dem Geräte-Host (111) zu empfangen und für den jeweiligen Geräte-Host (111) zu speichern und geänderte Konfigurationsprotokolle (130_A, 130_B) zu verteilen und einen zentralen Schreibzugriff auf die empfangenen und/oder gespeicherten Konfigurationsprotokolle (122, 124, 125) bereitzustellen; und
eine Administratoreinheit (130) mit einem flüchtigen und/oder nichtflüchtigen Speicher (131), einer Benutzerschnittstelle (132) und einem Prozessor (133) in operativer Kommunikation mit dem verteilten Datenbanksystem (120), wobei die Administratoreinheit (130) dazu konfiguriert ist, von dem verteilten Datenbanksystem (120) zumindest ein gespeichertes Konfigurationsprotokoll (122, 124, 125) für einen Geräte-Host (111) zu empfangen und lokal zu speichern und über die Benutzerschnittstelle (132) ein geändertes Konfigurationsprotokoll (133_A, 133_B) zu erzeugen und das geänderte Konfigurationsprotokoll (133_A, 133_B) in Antwort auf einen Befehl an das verteilte Datenbanksystem (120) zur Speicherung und/oder Verteilung an wenigstens ein ausgewähltes bildgebendes Gerät (110) zu senden.

2. System (100) nach Anspruch 1, wobei das bildgebende Gerät (110) ein Magnetresonanztomographie-Gerät (MRT-Gerät) oder ein Computertomografie-Gerät (CT-Gerät) umfasst, welches spezifische Eigenschaften aufweist, die für das Verteilen der geänderte Konfigurationsprotokolle (133_A, 133_B) durch das verteilte Datenbanksystem (120) an ein ausgewähltes bildgebendes Gerät (110) verwendet werden.

3. System (100) nach einem der vorhergehenden Ansprüche, wobei das bildgebende Gerät (110) sein Konfigurationsprotokoll (122, 124, 125) gespiegelt an das verteilte Datenbanksystem (120) sendet, insbesondere automatisiert sendet.

4. System (100) nach einem der vorhergehenden Ansprüche, wobei das verteilte Datenbanksystem (120) eine strukturierte Verzeichnisstruktur mit einem Unterverzeichnis (126, 127) für jede spezifische Software-Version der Geräte-Software auf dem Geräte-Host (111) der bildgebenden Geräte (110) umfasst und bildgebenden Geräte (110) mit der gleichen Software-Version in dem gleichen Unterverzeichnis (126, 127) gruppiert werden.

5. System (100) nach Anspruch 4, wobei für jede spezifische Software-Version der Geräte-Software eine zentrale Datenbank (121, 123) in einem spezifischen Unterverzeichnis (126, 127) des verteilten Datenbanksystems (120) entsprechend ihrer Software-Version eingerichtet ist; und/oder
wobei der Name des Unterverzeichnisses (126, 127) aus der Major-Version der ausgeführten Geräte-Software und einem Iterationswert bestimmt ist.

6. System (100) nach einem der Ansprüche 4 und 5, wobei in jedem softwareversionsspezifischen Unterverzeichnis eine Metadaten-Datei eingerichtet ist, welche entsprechende Informationen über die Software-Version umfasst.

7. System (100) nach einem der vorhergehenden Ansprüche, wobei geänderte Konfigurationsprotokolle (133_A, 133_B) automatisiert an die Geräte-Hosts (111) der bildgebenden Geräte (110) verteilt werden.

8. System (100) nach Anspruch 7, wobei die geänderten Konfigurationsprotokolle (133_A, 133_B) instantan und/oder nach einem vordefinierten Kriterium automatisiert an die Geräte-Hosts (111) der bildgebenden Geräte (110) verteilt werden.

9. System (100) nach einem der vorhergehenden Ansprüche, wobei ein für das bildgebende Gerät (110) geänderte Konfigurationsprotokoll (133_A, 133_B) nur für dieses bildgebende Gerät (110) sichtbar und/oder zugreifbar ist.

10. System (100) nach einem der vorhergehenden Ansprüche, wobei die in dem verteilten Datenbanksystem (120) abgespeicherten Daten zugriffsgeschützt abgespeichert sind.

11. System (100) nach einem der vorhergehenden Ansprüche, wobei die Administratoreinheit (130) ausgebildet ist, über den Prozessor (133) eine Validierungsalgorithmus zur Validierung der geänderten Konfigurationsprotokolle (133_A, 133_B) auszuführen.

12. System (100) nach Anspruch 11, wobei die geänderten Konfigurationsprotokolle (133_A, 133_B) gegenüber spezifischen Eigenschaften des bildgebenden Gerätes (110) validiert sind.

13. System (100) nach einem der vorhergehenden Ansprüche, wobei die Administratoreinheit (130) ausgebildet ist, die Konfigurationsprotokolle (122, 124, 125) über die Benutzerschnittstelle (132) lokal zu ändern und bei fehlender operativer Kommunikation mit dem verteilten Datenbanksystem (120) lokal zu speichern und erst bei operativer Kommunikation automatisiert an das verteilte Datenbanksystem (120) zu senden.

14. Computerimplementiertes Verfahren (10) zur Administration von bildgebenden Geräten (110) mit den Schritten:
- Senden (S1) eines auf einem Geräte-Host (111) eines bildgebenden Gerätes (110) installierten Konfigurationsprotokolls (122, 124, 125) an ein verteiltes Datenbanksystem (120);
- Empfangen (S2) des gesendeten Konfigurationsprotokolls (122) und Speichern (S3) des empfangenen Konfigurationsprotokolls (122, 124, 125) für den Geräte-Host (111) durch das verteilte Datenbanksystem (120);
- Bereitstellen (S4) des gespeicherten Konfigurationsprotokolls (122, 124, 125) für eine Administratoreinheit (130) zum Erzeugen eines geänderten Konfigurationsprotokolls (130_A, 130_B) mittels einer Anwendungssoftware, die auf der Administratoreinheit (130) ausgeführt wird; und
- Empfangen (S5) eines Befehls über die Anwendungssoftware und Senden (S6) des geänderten Konfigurationsprotokolls (130_A, 130_B) in Antwort auf den empfangenen Befehl an das verteilte Datenbanksystem (120) zur Speicherung und/oder Verteilung (S7) an wenigstens ein ausgewähltes bildgebendes Gerät (110).

15. Computerprogramm mit Programmcode für das Ausführen eines Verfahrens nach Anspruch 14, wenn das Computerprogramm auf einem elektronischen Gerät (130) ausgeführt wird.
